(19) Europäisches Patentamt
European Patent Office
Office européen des brevets

(11) Veröffentlichungsnummer: 0 123 113
B1

(12) EUROPÄISCHE PATENTSCHRIFT

(45) Veröffentlichungstag der Patentschrift: **28.09.88**

(21) Anmeldenummer: **84102904.4**

(22) Anmeldetag: **16.03.84**

(51) Int. Cl.⁴: **C 07 D 335/06,** C 07 D 311/30, C 07 D 311/22, C 07 D 311/24, C 07 D 405/04, C 07 D 409/04

# (54) Chromon- und Thiochromon-8-aldehyde, sowie ein Verfahren zu ihrer Herstellung.

(30) Priorität: **25.03.83 DE 3311004**

(43) Veröffentlichungstag der Anmeldung: **31.10.84 Patentblatt 84/44**

(45) Bekanntmachung des Hinweises auf die Patenterteilung: **28.09.88 Patentblatt 88/39**

(84) Benannte Vertragsstaaten: **AT BE CH DE FR GB IT LI LU NL SE**

(56) Entgegenhaltungen: **EP-A-0 080 934**

**CHEMICAL ABSTRACTS, Band 54, 1960, Spalte 21071b-e, Columbus, Ohio, US; S.M. PARIKH u.a.: "Fries transformation and Friedel-Crafts reaction. III. Friedel-Crafts acylation of some hydroxychromones" & J. INDIAN CHEM. SOC. 36, 841-4(1959)**

(73) Patentinhaber: **BAYER AG Konzernverwaltung RP Patentabteilung D-5090 Leverkusen 1 Bayerwerk (DE)**

(72) Erfinder: **Goldmann, Siegfried, Dr. Kuckuckstrasse 41 D-5600 Wuppertal 2 (DE)**

EP 0 123 113 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die vorliegende Erfindung betrifft Thiochromon-8-aldehyde sowie ein Verfahren zu ihrer Herstellung.

Thiochromone der Struktur I sind durch Kondensation von Thiophenolen mit Benzoylessigestern in Polyphosphorsäure zugänglich (*Bossert*, Lieb. Ann. *680*, 40 (1964)).

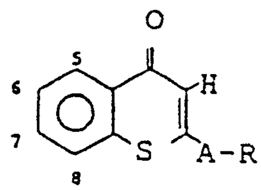

(I)

Nicht zugänglich auf diesem Wege sind die entsprechenden 8-Formylderivate.

Die vorliegende Erfindung betrifft Thiochromon-8-aldehyde der allgemeinen Formel (II)

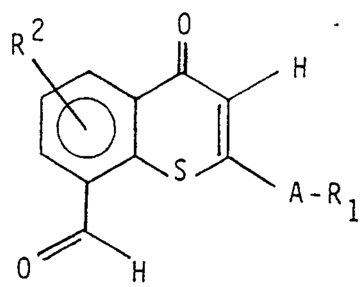

in denen

$R_1$ für Wasserstoff, einen $C_1$—$C_8$ aliphatischen Kohlenwasserstoffrest, einen $C_1$—$C_8$ Carbonsäurealkylesterrest, einen gegebenenfalls 1—5 fach gleich oder verschieden durch Fluor, Chlor, Brom, Jod, $C_1$—$C_8$ Alkyl, $C_1$—$C_8$ Alkylthio, $C_1$—$C_8$ Alkylsulfinyl, Cyano, Hydroxy, Nitro, $C_1$—$C_4$ Mono- oder Polyfluoralkyl, $C_1$—$C_4$ Mono- oder Polyfluoralkoxy, $C_1$—$C_4$ Mono- oder Polyfluoralkylthio, Amino, $C_1$—$C_5$ Monoalkylamino, $C_1$—$C_5$-Dialkylamino substituierten Rest aus der Gruppe Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht,

$R_2$ für Wasserstoff oder ein bis drei Fluor- oder Chloratome steht und

A für eine einfache Bindung oder eine $C_1$—$C_{18}$-Alkylenkette steht, die gegebenenfalls durch O oder S unterbrochen sein kann.

Von besonderem Interesse sind Verbindungen der allgemeinen Formel II, in denen

$R_1$ für Wasserstoff, einen $C_1$—$C_7$ aliphatischen Kohlenwasserstoffrest, einen $C_1$—$C_6$-Carbonsäurealkylester, einen gegebenenfalls ein- bis vierfach gleich oder verschieden durch Fluor, Chlor, Brom, $C_1$—$C_6$-Alkyl, $C_1$—$C_6$-Alkylthio, $C_1$—$C_6$-Alkylsulfinyl, Cyano, Hydroxy, Nitro, $C_1$—$C_3$-Mono- oder Polyfluoroalkyl, $C_1$—$C_3$-Mono- oder Polyfluoralkoxy, $C_1$—$C_3$-Mono- oder Polyfluoralkylthio, Amino, $C_1$—$C_4$-Monoalkylamino, $C_1$—$C_4$-Dialkylamino, substituierten Rest aus der Gruppe Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, Benzimidazolyl oder Chinazolyl steht,

$R_2$ für Wasserstoff oder 1—3 Fluoratome steht,

A für eine Einfachbindung oder einen $C_1$—$C_{16}$-Alkylenrest steht, der gegebenenfalls durch O oder S unterbrochen sein kann.

Insbesondere sei Thioflavon-8-carboxaldehyd erwähnt.

Die Erfindung betrifft weiterhin ein Verfahren zur Herstellung von Verbindungen der allgemeinen Formel (II), das dadurch gekennzeichnet ist, daß man Thiochromone der allgemeinen Formel (III)

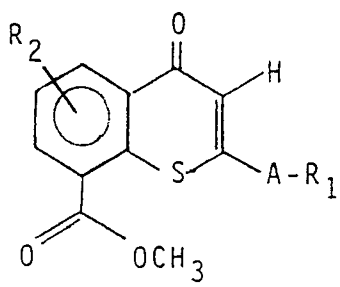

(III),

in welcher

$R^1$, $R^2$ und A die oben angegebene Bedeutung haben, zu Benzylalkoholen der allgemeinen Struktur (IV) reduziert und daß man den Benzylalkohol (IV)

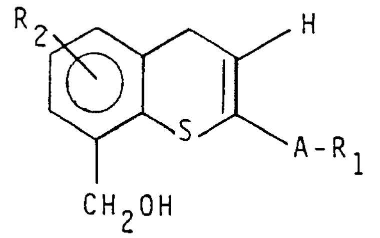

(IV)

mit Oxidationsmitteln zu Aldehyden der Formel (II) oxidiert.

Die als Ausgangsstoffe verwendeten Thioflavone sind bekannt oder können nach bekannten Verfahren hergestellt werden (*Bossert*, Lieb. Ann. *680*, 40 (1964)).

Für die Reduktion zum Benzylalkohol (IV) können inerte organische Lösungsmittel eingesetzt werden. Beispielsweise Ether, wie z.B. Dioxan, Diethylether, Tetrahydrofuran, Dimethoxyethan oder Aromaten wie z.B. Toluol oder Benzol. Als Reduktionsmittel seien beispielhaft Alkalialuminiumhydride wie z.B. $LiAlH_4$ oder Alkylaluminiumhydride wie z.B. Diisobutylaluminiumhydrid erwähnt.

Bei der Durchführung dieses erfindungsgemäßen Verfahrens arbeitet man vorzugsweise in einem Temperaturbereich von −100°C bis +60°C, insbesondere in einem Bereich von −60°C bis +30°C.

Die Umsetzung erfolgt üblicherweise bei Normaldruck, kann aber auch bei erhöhtem Druck durchgeführt werden.

Das Reduktionsmittel wird in dem Fachmann geläufigen Mengen zugesetzt, bevorzugt in mindestens vier und höchstens 8 Hydridäquivalenten.

Für die Oxidation des Benzylalkohols (IV) zum Aldehyd (II) können die gleichen Lösungsmittel wie bei der Reduktion Verwendung finden, zusätzlich aber auch halogenierte Kohlenwasserstoffe wie Chloroform und Methylenchlorid oder Ketone wie z.B. Aceton.

Als Oxidationsmittel können die üblicherweise für Oxidationen eingesetzten Übergangsmetalloxide eingesetzt werden, vorzugsweise jedoch Mangandioxid.

Bei der Durchführung der Oxidation arbeitet man normalerweise in einem Temperaturbereich von −30 bis +200°C vorzugsweise beim Siedepunkt des jeweiligen Lösungsmittels. Die Oxidation erfolgt üblicherweise bei Normaldruck, kann aber auch bei erhöhtem Druck durchgeführt werden.

Das Oxidationsmittel kann in 3 bis 20, vorzugsweise in 5 bis 10 Oxidationsäquivalenten eingesetzt werden. Auch kann es zweckmäßig sein, von Zeit zu Zeit frisches Oxidationsmittel zum Reaktionsansatz zuzugeben.

Verwendet man Thioflavon-8-carbonsäuremethylester als Ausgangsstoff, so kann der Reaktionsablauf durch das folgende Formelschema wiedergegeben werden:

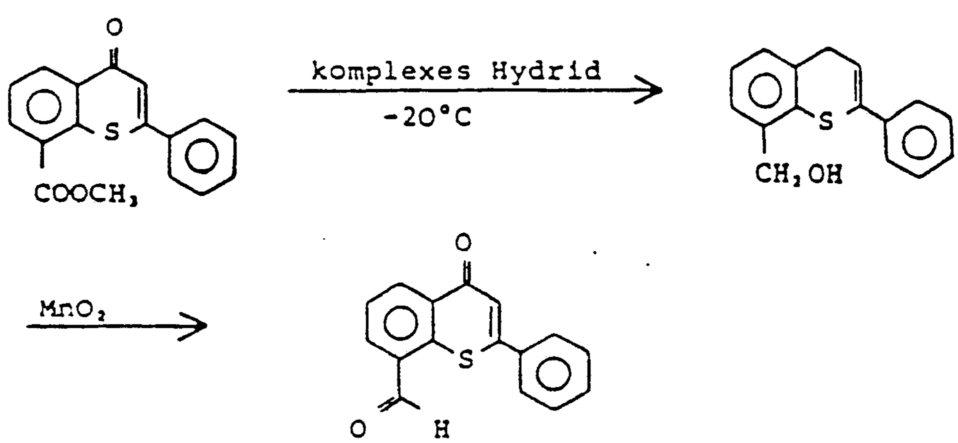


Die erfindungsgemäßen Verbindungen der Formel (II) lassen sich auf einfache Weise nach bekannten Methoden in die 1,4-Dihydropyridine überführen (*A. Hantzsch*, Just. Lieb. Ann. Chem. *215*, 1, 1882, übersicht: *U. Eisner, J. Kuthan*, Chem. Rev. *72*, 1 (1972)).

Diese 1,4-Dihydropyridine stellen wertvolle pharmazeutisch wirksame Verbindungen dar, die zur Behandlung von Kreislauferkrankungen eingesetzt werden können.

Das erfindungsgemäße Verfahren und die so hergestellten neuen Verbindungen stellen somit einen vorteilhaften Weg dar zur Herstellung von neuen pharmazeutisch wirksamen Stoffen.

Die nachfolgenden Beispiele sollen das erfindungsgemäße Verfahren erläutern, ohne es einzuschränken.

Beispiel 1

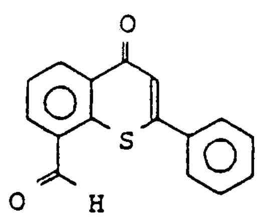

50 g Thioflavon-8-carbonsäuremethylester werden in Tetrahydrofuran bei 0°C mit 4 eq. Diisobutylaluminiumhydrid (in Toluol) versetzt, nach Hydrolyse mit verdünnter Schwefelsäure mit Ether extrahiert, getrocknet und eingedampft.

Man erhält 80% 8-Hydroxymethyl-2-phenyl-4H-thiochromen (Fp: 120—122°C).

20 g 8-Hydroxymethyl-2-phenyl-4H-thiochromen werden in 500 ml Chloroform gelöst und mit 5 eq. Mangandioxid 10 Stunden am Rückfluß erhitzt, abgesaugt und eingeengt. Man erhielt 60% Thioflavon-8-carboxaldehyde (Fp: 153—157°C).

$^1$H—NMR ($CDCl_3$) $\delta$ = 7,3 (s. 1H), 7.45—7.6 (m, 3H) 7.7—7.8 (m, 3H), 8.2 (d, J = 7 Hz, 1H), 8.9 (d, J = 7 Hz, 1H), 10,25 (s, 1H).

Analog Beispiel 1 wurde dargestellt

Beispiel 2

Fp: 208—210°C

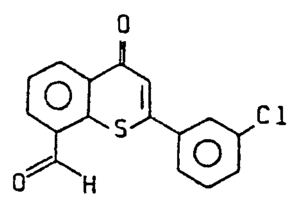

**Patentansprüche**

1. Verbindungen der Formel

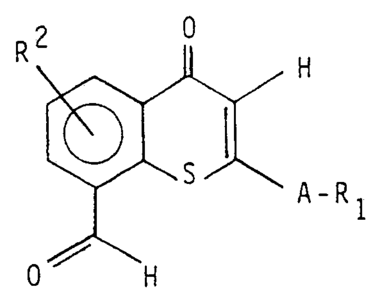

in denen

$R_1$ für Wasserstoff, einen $C_1$—$C_8$ aliphatischen Kohlenwasserstoffrest, einen $C_1$—$C_8$ Carbonsäurealkylesterrest, einen gegebenenfalls 1—5 fach gleich oder verschieden durch Fluor, Chlor, Brom, Jod, $C_1$—$C_8$ Alkyl, $C_1$—$C_8$ Alkylthio, $C_1$—$C_8$ Alkylsulfinyl, Cyano, Hydroxy, Nitro, $C_1$—$C_4$ Mono- oder Polyfluoralkyl, $C_1$—$C_4$ Mono- oder Polyfluoralkoxy, $C_1$—$C_4$ Mono- oder Polyfluoralkylthio, Amino, $C_1$—$C_5$ Monoalkylamino, $C_1$—$C_5$-Dialkylamino substituierten Rest aus der Gruppe Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht,

$R_2$ für Wasserstoff oder ein bis drei Fluor- oder Chloratome steht und

A für eine einfache Bindung oder eine $C_1$—$C_{18}$-Alkylenkette steht, die gegebenenfalls durch O oder S unterbrochen sein kann.

2. Verbindungen gemäß Anspruch 1, in denen

$R_1$ für Wasserstoff, einen $C_1$—$C_7$ aliphatischen Kohlenwasserstoffrest, einen $C_1$—$C_6$-Carbonsäurealkylester, einen gegebenenfalls 1—4 fach gleich oder verschieden durch Fluor, Chlor, Brom,

$C_1$—$C_6$-Alkyl, $C_1$—$C_6$-Alkylthio, $C_1$—$C_6$-Alkylsulfinyl, Cyano, Hydroxy, Nitro, $C_1$—$C_3$-Mono- oder Polyfluoroalkyl, $C_1$—$C_3$ Mono- oder Polyfluoralkoxy, $C_1$—$C_3$ Mono- oder Polyfluoralkylthio, Amino, $C_1$—$C_4$ Monoalkylamino, $C_1$—$C_4$ Dialkylamino, substituierten Rest aus der Gruppe Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, Benzimidazolyl oder Chinazolyl steht,

$R_2$ für Wasserstoff oder 1—3 Fluoratome steht,

A für eine Einfachbindung oder einen $C_1$—$C_{16}$-Alkylenrest steht, der gegebenenfalls durch O oder S unterbrochen sein kann.

3. Thioflavon-8-carboxaldehyd.

4. Verfahren zur Herstellung von Verbindungen der Formel

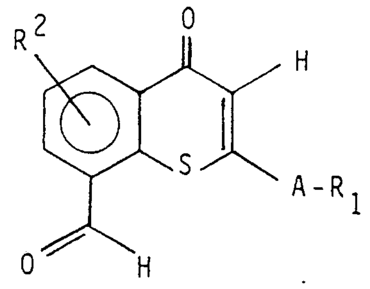

in der

$R_1$ für Wasserstoff, einen $C_1$—$C_8$ aliphatischen Kohlenwasserstoffrest, einen $C_1$—$C_8$ Carbonsäurealkylesterrest, einen gegebenenfalls 1—5 fach gleich oder verschieden durch Fluor, Chlor, Brom, Jod, $C_1$—$C_8$ Alkyl, $C_1$—$C_8$ Alkylthio, $C_1$—$C_8$ Alkylsulfinyl, Cyano, Hydroxy, Nitro, $C_1$—$C_4$ Mono- oder Polyfluoralkyl, $C_1$—$C_4$ Mono- oder Polyfluoralkoxy, $C_1$—$C_4$ Mono- oder Polyfluoralkylthio, Amino, $C_1$—$C_5$ Monoalkylamino, $C_1$—$C_5$-Dialkylamino substituierten Rest aus der Gruppe Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht,

$R_2$ für Wasserstoff oder ein bis drei Fluor- oder Chloratome steht und

A für eine einfache Bindung oder eine $C_1$—$C_{18}$-Alkylenkette steht, die gegebenenfalls durch O oder S unterbrochen sein kann,

stehen, dadurch gekennzeichnet, daß man Thiochromone der Formel

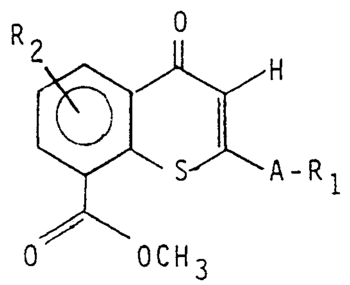

(III),

zu den entsprechenden Benzylalkoholen der Formel

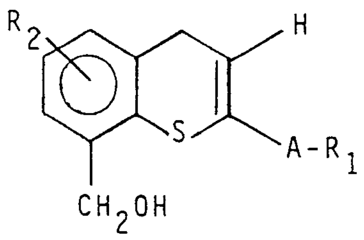

(IV)

reduziert und anschließend mit Oxidationsmitteln zu den Aldehyden oxidiert.

5. Verfahren nach Anspruch 4 zur Herstellung von Verbindungen, in denen

$R_1$ für Wasserstoff, einen $C_1$—$C_7$ aliphatischen Kohlenwasserstoffrest, einen $C_1$—$C_6$-Carbonsäurealkylester, einen gegebenenfalls 1—4 fach gleich oder verschieden durch Fluor, Chlor, Brom, $C_1$—$C_6$-Alkyl, $C_1$—$C_6$-Alkylthio, $C_1$—$C_6$-Alkylsulfinyl, Cyano, Hydroxy, Nitro, $C_1$—$C_3$-Mono- oder

Polyfluoroalkyl, $C_1$—$C_3$ Mono- oder Polyfluoralkoxy, $C_1$—$C_3$ Mono- oder Polyfluoralkylthio, Amino, $C_1$—$C_4$ Monoalkylamino, $C_1$—$C_4$ Dialkylamino, substituierten Rest aus der Gruppe Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, Benzimidazolyl oder Chinazolyl steht,

$R_2$ für Wasserstoff oder 1—3 Fluoratome steht,

A für eine Einfachbindung oder einen $C_1$—$C_{16}$-Alkylenrest steht, der gegebenenfalls durch O oder S unterbrochen sein kann.

6. Verfahren nach Anspruch 4 zur Herstellung von Thioflavon-8-carboxaldehyd.

7. Verfahren nach Anspruch 4, dadurch gekennzeichnet, daß man als Reduktionsmittel Alkalialuminiumhydride oder Alkylaluminiumhydride einsetzt.

8. Verfahren nach Anspruch 4, dadurch gekennzeichnet, daß man die Reduktion bei Temperaturen von −100°C bis 60°C und die Oxidation bei Temperaturen von −30°C bis 200°C durchführt.

9. Verfahren nach Anspruch 4, dadurch gekennzeichnet, daß man als Oxidationsmittel Mangandioxid einsetzt.

**Revendications**

1. Composés de formule

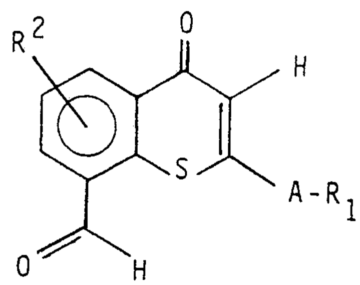

dans lesquels

$R^1$ représente l'hydrogène, un reste d'hydrocarbure aliphatique en $C_1$ à $C_8$, un reste d'ester alkylique d'acide carboxylique en $C_1$ à $C_8$, un reste du groupe phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, isoquinolyle, indolyle, benzimidazolyle, quinazolyle ou quinoxalyle portant éventuellement 1 à 5 substituants, identiques ou différents, fluoro, chloro, bromo, iodo, alkyle en $C_1$ à $C_8$, alkylthio en $C_1$ à $C_8$, alkylsulfinyle en $C_1$ à $C_8$, cyano, hydroxy, nitro, mono- ou poly-(fluoralkyle en $C_1$ à $C_4$), mono- ou poly-(fluoralkoxy en $C_1$ à $C_4$), mono- ou poly(fluoralkylthio en $C_1$ à $C_4$), amino, monoalkylamino en $C_1$ à $C_5$, di(alkyle en $C_1$ à $C_5$)amino,

$R^2$ représente l'hydrogène ou 1 à 3 atomes de fluor ou de chlore et

A est une liaison simple ou une chaîne alkylénique en $C_1$ à $C_{18}$ qui peut éventuellement être interrompue par de l'oxygène ou du soufre.

2. Composés suivant la revendication 1, dans lesquels

$R_1$ représente l'hydrogène, un reste d'hydrocarbure aliphatique en $C_1$ à $C_7$, un ester alkylique d'acide carboxylique en $C_1$ à $C_6$, un reste du groupe phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle, benzimidazolyle ou quinazolyle portant éventuellement 1 à 4 substituants, identiques ou différents, fluoro, chloro, bromo, alkyle en $C_1$ à $C_6$, alkylthio en $C_1$ à $C_6$, alkylsulfinyle en $C_1$ à $C_6$, cyano, hydroxy, nitro, mono- ou poly-(fluoralkyle en $C_1$ à $C_3$), mono- ou poly-(fluoralkoxy en $C_1$ à $C_3$), mono- ou poly-(fluoralkylthio en $C_1$ à $C_3$), amino, monoalkylamino en $C_1$ à $C_4$, di(alkyle en $C_1$ à $C_4$)amino,

$R_2$ représente l'hydrogène ou 1 à 3 atomes de fluor,

A est une liaison simple ou un reste alkylène en $C_1$ à $C_{16}$ qui peut éventuellement être interrompue par de l'oxygène ou du soufre.

3. Le thioflavone-8-carboxaldéhyde.

4. Procédé de production de composés de formule

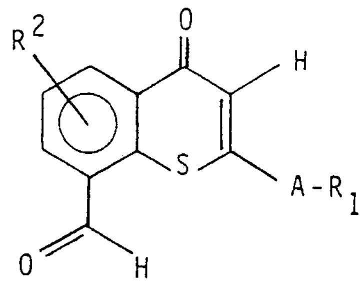

dans laquelle

$R^1$ représente l'hydrogène, un reste d'hydrocarbure aliphatique en $C_1$ à $C_8$, un reste d'ester alkylique d'acide carboxylique en $C_1$ à $C_8$, un reste du groupe phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, isoquinolyle, indolyle, benzimidazolyle, quinazolyle ou quinoxalyle portant éventuellement 1 à 5 substituants, identiques ou différents, fluoro, chloro, bromo, iodo, alkyle en $C_1$ à $C_8$, alkylthio en $C_1$ à $C_8$, alkylsulfinyle en $C_1$ à $C_8$, cyano, hydroxy, nitro, mono- ou poly-(fluoralkyle en $C_1$ à $C_4$), mono- ou poly-(fluoralkoxy en $C_1$ à $C_4$), mono- ou poly(fluoralkylthio en $C_1$ à $C_4$), amino, monoalkylamino en $C_1$ à $C_5$, di(alkyle en $C_1$ à $C_5$)amino,

$R^2$ représente l'hydrogène ou 1 à 3 atomes de fluor ou de chlore et

A est une liaison simple ou une chaîne alkylénique en $C_1$ à $C_{18}$ qui peut éventuellement être interrompue par de l'oxygène ou du soufre.

caractérisé en ce qu'on réduit des thiochromones de formule

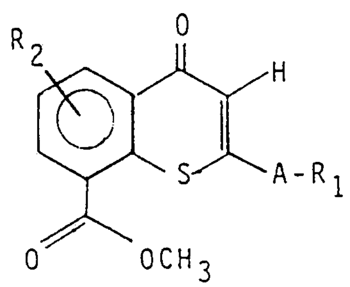

(III),

en les alcools benzyliques correspondants de formule

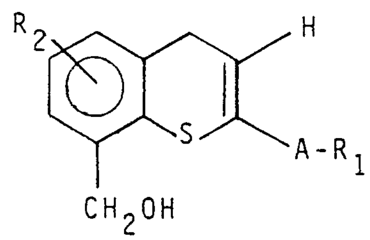

(IV)

que l'on oxyde ensuite en les aldéhydes avec des agents oxydants.

5. Procédé suivant la revendication 4 pour l'obtention de composés dans lesquels

$R_1$ représente l'hydrogène, un reste d'hydrocarbure aliphatique en $C_1$ à $C_7$, un ester alkylique d'acide carboxylique en $C_1$ à $C_6$, un reste du groupe phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle, benzimidazolyle ou quinazolyle portant éventuellement 1 à 4 substituants, identiques ou différents, fluoro, chloro, bromo, alkyle en $C_1$ à $C_6$, alkylthio en $C_1$ à $C_6$, alkylsulfinyle en $C_1$ à $C_6$, cyano, hydroxy, nitro, mono- ou poly(fluoralkyle en $C_1$ à $C_3$), mono- ou poly-(fluoralkoxy en $C_1$ à $C_3$), mono- ou poly-(fluoralkylthio en $C_1$ à $C_3$), amino, monoalkylamino en $C_1$ à $C_4$, di(alkyle en $C_1$ à $C_4$)amino,

$R_2$ représente l'hydrogène ou 1 à 3 atomes de fluor,

A est une liaison simple ou un reste alkylène en $C_1$ à $C_{16}$ qui peut éventuellement être interrompu par de l'oxygène ou du soufre.

6. Procédé suivant la revendication 4 pour la préparation du thioflavone-8-carboxaldéhyde.

7. Procédé suivant la revendication 4, caractérisé en ce qu'on utilise comme agent réducteur des hydrures de métaux alcalins et d'aluminium ou des hydrures d'alkylaluminium.

8. Procédé suivant la revendication 4, caractérisé en ce qu'on conduit la réduction à des températures de −100°C à 60°C et on conduit l'oxydation à des températures de −30°C à 200°C.

9. Procédé suivant la revendication 4, caractérisé en ce qu'on utilise le dioxyde de manganèse comr agent oxydant.

**Claims**

1. Compounds of the formula

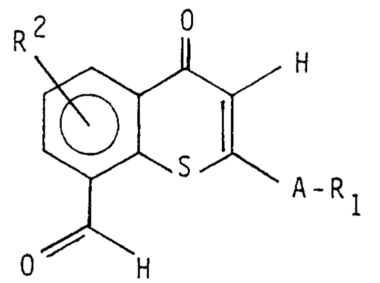

in which

$R_1$ represents hydrogen, a $C_1$—$C_8$ aliphatic hydrocarbon radical, a $C_1$—$C_8$ alkyl carboxylate, or a radical from the group comprising phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl or quinoxalyl which optionally has 1—5 identical or different fluorine, chlorine, bromine, iodine, $C_1$—$C_8$ alkyl, $C_1$—$C_8$ alkylthio, $C_1$—$C_8$ alkylsulphinyl, cyano, hydroxyl, nitro, $C_1$—$C_4$ monofluoroalkyl or polyfluoroalkyl, $C_1$—$C_4$ monofluoroalkoxy or polyfluoroalkoxy, $C_1$—$C_4$ monofluoroalkylthio or polyfluoroalkylthio, amino, $C_1$—$C_5$ monoalkylamino or $C_1$—$C_5$-dialkylamino substituents,

$R_2$ represents hydrogen or one to three fluorine or chlorine atoms, and

A represents a single bond or a $C_1$—$C_{18}$-alkylene chain which can optionally be interrupted by O or S.

2. Compounds according to Claim 1, in which

$R_1$ represents hydrogen, a $C_1$—$C_7$ aliphatic hydrocarbon radical, a $C_1$—$C_6$ alkyl carboxylate, or a radical from the group comprising phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl, benzimidazolyl or quinazolyl which optionally has 1—4 identical or different fluorine, chlorine, bromine, $C_1$—$C_6$ alkyl, $C_1$—$C_6$ alkylthio, $C_1$—$C_6$ alkylsulphinyl, cyano, hydroxyl, nitro, $C_1$—$C_3$-monofluoroalkyl or -polyfluoroalkyl, $C_1$—$C_3$ monofluoroalkoxy or polyfluoroalkoxy, $C_1$—$C_3$ monofluoroalkylthio or polyfluoroalkylthio, amino, $C_1$—$C_4$ monoalkylamino or $C_1$—$C_4$ dialkylamino substituents,

$R_2$ represents hydrogen or 1—3 fluorine atoms, and

A represents a single bond or a $C_1$—$C_{16}$-alkylene radical which can optionally be interrupted by O or S.

3. Thioflavone-8-carboxaldehyde.

4. Process for the preparation of compounds of the formula

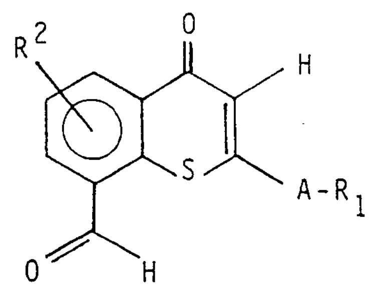

in which

$R_1$ represents hydrogen, a $C_1$—$C_8$ aliphatic hydrocarbon radical, a $C_1$—$C_8$ alkyl carboxylate, or a radical from the group comprising phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl or quinoxalyl which optionally has 1—5 identical or different fluorine, chlorine, bromine, iodine, $C_1$—$C_8$ alkyl, $C_1$—$C_8$ alkylthio, $C_1$—$C_8$ alkylsulphinyl, cyano, hydroxyl, nitro, $C_1$—$C_4$ monofluoroalkyl or polyfluoroalkyl, $C_1$—$C_4$ monofluoroalkoxy or polyfluoroalkoxy, $C_1$—$C_4$ monofluoroalkylthio or

polyfluoroalkylthio, amino, $C_1$—$C_5$ monoalkylamino or $C_1$—$C_5$-dialkylamino substituents,

$R_2$ represents hydrogen or one to three fluorine or chlorine atoms, and

A represents a single bond or a $C_1$—$C_{18}$-alkylene chain which can optionally be interrupted by O or S, characterised in that thiochromones of the formula

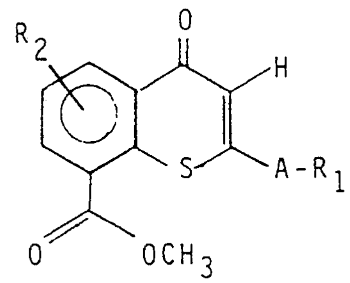

(III),

are reduced to give the corresponding benzyl alcohols of the formula

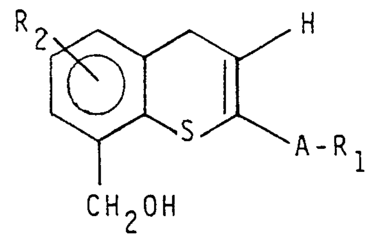

(IV)

and then oxidised with oxidising agents to give the aldehydes.

5. Process according to Claim 4 for the preparation of compounds in which

$R_1$ represents hydrogen, a $C_1$—$C_7$ aliphatic hydrocarbon radical, a $C_1$—$C_6$ alkyl carboxylate, or a radical from the group comprising phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl, benzimidazolyl or quinazolyl which optionally has 1—4 identical or different fluorine, chlorine, bromine, $C_1$—$C_6$ alkyl, $C_1$—$C_6$ alkylthio, $C_1$—$C_6$-alkylsulphinyl, cyano, hydroxyl, nitro, $C_1$—$C_3$-monofluoroalkyl or $C_1$—$C_3$-polyfluoroalkyl, $C_1$—$C_3$ monofluoroalkoxy or polyfluoroalkoxy, $C_1$—$C_3$ monofluoroalkylthio or polyfluoroalkylthio, amino, $C_1$—$C_4$ monoalkylamino or $C_1$—$C_4$ dialkylamino substituents,

$R_2$ represents hydrogen or 1—3 fluorine atoms, and

A represents a single bond or a $C_1$—$C_{16}$-alkylene radical which can optionally be interrupted by O or S.

6. Process according to Claim 4 for the preparation of thioflavone-8-carboxaldehyde.

7. Process according to Claim 4, characterised in that alkali metal aluminium hydrides or alkyl aluminium hydrides are employed as the reducing agent.

8. Process according to Claim 4, characterised in that the reduction is carried out at temperatures from −100°C to 60°C and the oxidation is carried out at temperatures from −30°C to 200°C.

9. Process according to Claim 4, characterised in that manganese dioxide is employed as the oxidising agent.